# EUROPEAN PATENT APPLICATION

(11) **EP 4 736 942 A1**
(43) Date of publication of application: **06.05.2026**
(21) Application number: 25213117.2
(22) Date of filing: 03.11.2025
(51) Int. Cl.: A61N 5/10, G01T 1/185

(54) **APPARATUS FOR MEASURING RADIOTHERAPY BEAM DOSE**

(30) Priority: 04.11.2024 CN 202411562182
(71) Applicant: Elekta Limited, Crawley, Sussex RH10 9RR (GB)
(72) Inventor: LI, Peng, Crawley (GB); ZHANG, Zhiquan, Crawley (GB); WANG, Chao, Crawley (GB); GENG, Xingren, Crawley (GB)
(74) Representative: Thomas, Tomos Daniel

(57) **Abstract**

Disclosed herein are apparatus, methods, systems, and/or computer-readable media for measuring, detecting, determining and/or monitoring radiotherapy beam dose of a radiotherapy device. The apparatus, methods, systems, and/or computer-readable media disclosed herein provide an approach that involves use of a pair of plates for generating a voltage between the pair of plates, and a beam detection volume arranged at least in part between the pair of plates.

## Description

This disclosure relates to apparatus, devices, systems, and approaches for radiotherapy, and in particular to apparatus, methods, and/or computer-readable media for measuring radiotherapy beam dose.

### Background

Radiotherapy can be described as the use of ionising radiation, such as X-rays, to treat a human or animal body. Radiotherapy is commonly used to treat tumours within the body of a human or animal patient, or subject. In such treatments, ionising radiation is used to irradiate, and thus destroy or damage, cells which form part of the tumour.

Radiotherapy systems are highly complex machines having a significant number of complex interacting subsystems. Many radiotherapy systems use a beam generation subsystem based on a particle accelerator such as a linear accelerator to produce a beam of ionising radiation. The measurement of beam energy or dose is important for ensuring radiotherapy is delivered accurately, effectively, and according to plan. Devices for providing such measurements must meet particular requirements set out in international standards. There is therefore a general need for improved approaches for detecting, measuring, and/or monitoring radiotherapy beam energy and/or dose in a radiotherapy system.

### Summary

An invention is set out in the independent claims. Optional features are set out in the dependent claims.

Examples will now be described, by way of example only, with reference to the drawings of which:
Fig. 1 shows a radiotherapy device or apparatus;
Fig. 2 shows apparatus for measuring radiotherapy beam dose according to the present disclosure;
Fig. 3A shows a perspective view of apparatus for measuring radiotherapy beam dose according to the present disclosure;
Fig. 3B shows another perspective view of apparatus for measuring radiotherapy beam dose according to the present disclosure;
Fig. 4 shows a flowchart of a method for measuring radiotherapy beam dose according to the present disclosure;
Fig. 5 shows a block diagram of one implementation of a radiotherapy system; and
Fig. 6 shows a computer readable medium or, more generally, a computer program product.

### Detailed Description

Disclosed herein are systems, devices, methods and apparatuses relating to radiotherapy. With linear accelerator-based radiotherapy devices being highly complex and having many inter-related parts, the terms "system", "device", "apparatus", "subsystem", and "machine" may all be applied interchangeably to describe the radiotherapy apparatus as a whole, or collections of components of the radiotherapy apparatus. The term "apparatus" as used herein may refer to either a single apparatus or plural apparatus and should not be understood as being particularly limited to either a single discrete apparatus or a plurality of discrete apparatus unless a particular apparatus is further described as such.

The term "treatment beam" is used herein, but should not be taken to always correspond to a beam of radiation that is necessarily being used for treating a patient. For example, the "treatment" beam discussed herein may be a beam of ionising radiation that is produced by a radiotherapy system during calibration, installation, and/or set-up of the radiotherapy system in the absence of any patient.

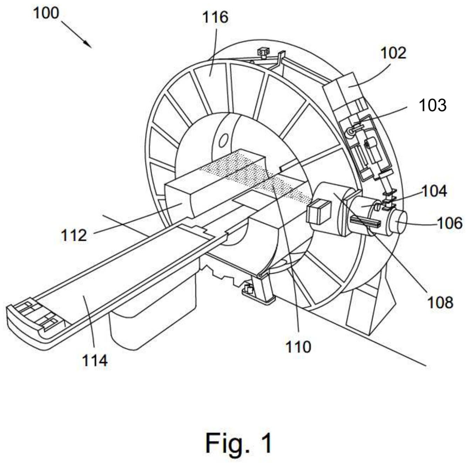

Fig. 1 shows an exemplary radiotherapy (RT) system or device 100. The device 100 and its constituent components will be well known to the skilled person but is described here generally for the purpose of providing useful accompanying information for the present disclosure. The radiotherapy device 100 is based on a linear accelerator (linac).

The device 100 shown in Fig. 1 combines magnetic resonance (MR) imaging capability with a linac-based radiotherapy capability, and is known as an MR-linac device. However, the present disclosure may be implemented in any radiotherapy device, for example, a linac-based radiotherapy device without magnetic resonance imaging capability. In operation, the MR scanner produces MR images of the patient, and the RT apparatus produces and shapes a beam of radiation and directs it toward a target region within a patient's body in accordance with a radiotherapy treatment plan.

The MR-linac device 100 shown in Fig. 1 comprises an RF power source 102, an RF transmission apparatus 103, an acceleration waveguide 104, an electron source 106, a treatment head including a collimator 108 such as a multi-leaf collimator used to shape a treatment beam 110, MR imaging apparatus 112 (shown partially cut away), and a patient support surface 114. The RF transmission apparatus 103 comprises a waveguide component, which may be a copper waveguide. The depicted device 100 does not have the usual 'housing' which would cover the MR imaging apparatus and RT apparatus in a commercial setting such as a hospital. In use, the device 100 would also comprise the housing, part of which, together with the ring-shaped gantry, defines a bore. In particular, a part of the housing encloses the inner surface of the ring-shaped gantry, defining a bore through the device 100. The patient support surface 114 is moveable and can be used to support a patient and move them, or another subject, into the bore when an MR scan and/or when radiotherapy is to commence.

The MR imaging apparatus 112 is configured to obtain images of a subject positioned on the patient support surface 114. The MR imaging apparatus 112 may be conventional MR imaging apparatus operating in a known manner to obtain MR data, for example MR images. The skilled person will appreciate that such MR imaging apparatus 112 may comprise a primary magnet, one or more gradient coils, one or more receive coils, and an RF pulse applicator.

The RT device 100 has a beam generation subsystem comprising the RF power source 102, the acceleration waveguide 104, and the electron source 106. The beam generation subsystem is configured to produce a beam of ionising radiation, otherwise known as the treatment beam 110, that is collimated and shaped by the collimator 108 and directed towards the bore. Typically, a radiation detector is positioned diametrically opposed to the collimator. The radiation detector is suitable for, and configured to, produce radiation intensity data. In particular, the radiation detector is positioned and configured to detect the intensity of radiation which has passed through the subject. The radiation detector may form part of a portal imaging system.

The beam generation subsystem is attached to the rotatable gantry 116 so as to rotate with the gantry 116. In this way, the beam generation system is rotatable around the patient so that the treatment beam 110 can be applied from different angles around the gantry 116. In a preferred implementation, the gantry is continuously rotatable. In other words, the gantry can be rotated by 360 degrees around the patient, and in fact may continue to be rotated past 360 degrees. The gantry is ring-shaped, i.e. a ring-gantry.

The device 100 of Fig. 1 is controlled by a controller (not shown). The controller is a computer, processor, or other processing apparatus. The controller may be formed by several discrete processors; for example, the controller may comprise an MR imaging apparatus processor, which controls the MR imaging apparatus 112; an beam generation subsystem processor, which controls the operation of the beam generation subsystem; and a subject support surface processor which controls the operation and actuation of the patient support surface 114. The controller is communicatively coupled to a memory, e.g. a computer readable medium.

The present disclosure discloses apparatus for determining, measuring and/or monitoring properties such as energy and dose of a treatment beam such as the treatment beam 116 in Fig. 1. The apparatus disclosed herein may be a dosimetry apparatus.

Conventional approaches for radiotherapy dosimetry may be based on an ion chamber. Ion chambers operate by converting incoming radiation into electrons and then measuring the current produced by the electrons to derive the beam energy. In particular, an ion chamber may be based on a gas that is ionisable by the radiation. Conventionally, electronics and electrical components are provided on the outside of the ion chamber to extract the current and provide an electrical interface to the ion chamber. The electronics and electrical components are provided on the outside of the ion chamber away from the beam path to reduce or avoid their exposure to radiation. The electronics and electrical components may include associated cabling and/or be mounted on a board and occupy a relatively large space in relation to the ion chamber.

The ion chamber is typically attached or mounted as part of the beam delivery assembly of the radiotherapy system, such as between the acceleration waveguide 104 and the collimator 108 in the treatment head of Fig. 1. For gantry-based radiotherapy, the size and/or weight of the treatment head and beam generation subsystem, and the layout of components therein, is an important factor in gantry performance, which in turn can determine the rate, speed and/or efficacy with which radiotherapy can be delivered to a patient. Furthermore, typically, radiotherapy systems are very large, and require a large volume of physical space in a clinical setting, where space (or system 'real estate') is costly. As new approaches for radiotherapy are developed, which may seek to take advantage of improved gantry performance and/or to reduce the overall size of the system, new approaches for the treatment head and beam delivery components are required. In particular, making those sub-systems more compact can facilitate improved approaches to radiotherapy, such as by providing a more lightweight or compact beam delivery subsystem.

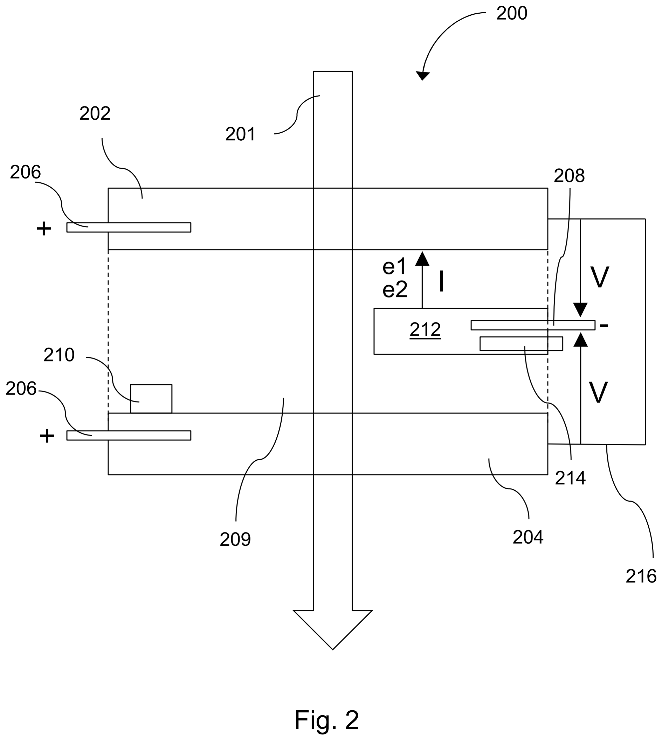

Fig. 2 shows an apparatus 200 for measuring radiotherapy beam dose according to the present disclosure. As shown in Fig. 2, a treatment beam 201 is passing through the apparatus 200. It will be appreciated that the treatment beam 201 may be like the treatment beam 110 of Fig. 1, and may be considered and/or referred to as a beam of ionising radiation, or a beam of therapeutic radiation, and/or a radiotherapy beam, and such like.

The apparatus 200 comprises a pair of plates 202, 204. Each of the pair of plates 202, 204 may comprise an electrically conductive material, such as metal, in order to enable them to each exhibit a potential difference or voltage with respect to a signal electrode 212. In some examples, each of the plates 202, 204 may be supplied with a positive voltage 206 and the signal electrode 212 may be supplied with a negative or ground voltage 208, as shown in Fig. 2, thereby creating a potential difference within a volume between the plates 202, 204. Any suitable terminal or connection may be used for providing voltage to each plate and/or the signal electrode. Likewise, the pair of plates 202, 204 and/or the signal electrode 212 may each take any suitable form for enabling the generation of the voltage, such as taking the form of metal electrodes. In some examples, the signal electrode 212 may take the form of a plate which may optionally be formed of metal, and/or be formed using another electrically conductive material, and/or take a different shape to that of a plate. It will be appreciated that, in some examples, the voltage direction may be reversed compared with that shown in Fig. 2. It will be appreciated that the pair of plates 202, 204 may be electrically connected to each other, such as through the connection 216 shown in Fig. 2, such that they can be held at the same voltage.

The pair of plates 202, 204 have opposing surfaces and a gap between them. In some examples, the pair of plates 202, 204 have flat opposing surfaces that are parallel to one another, however other arrangements are possible. For example, a cylindrical arrangement of each of plates 202, 204 may be used, wherein the opposing surfaces of each plate are curved and one plate is positioned within a hollow cylinder-like shape formed by the other plate.

The apparatus 200 further comprises a beam detection volume 209 arranged at least in part between the pair of plates (with side edges marked with dashed lines in Fig. 2). When a voltage is generated between the plates 202, 204 and the signal electrode 212, the beam detection volume 209 may at least in part be subject to an electric field corresponding to that voltage. It will be appreciated that in the example of Fig. 2, the signal electrode 212 laterally extends only partially into the beam detection volume 209, however, in other examples, the signal electrode 212 may extend fully across or across most of the beam detection volume 209 such that it spans the beam detection volume 209 in a direction parallel to the opposing surfaces of the two plates 202, 204. In some examples, the signal electrode 212 may be considered to be a middle plate between the pair of plates 202, 204.

The beam detection volume 209 is arranged to interact with the treatment beam 201 to determine a property of the treatment beam 201, such as energy or dose. For example, the beam detection volume 209 may be formed in a manner similar to an ion chamber and filled with ionisable gas such that when the treatment beam 201 passes through it, ion pairs are created (with examples shown as e1, e2 in Fig. 2). Under the influence of the electric field associated with the voltage difference between the pair of plates 202, 204 and the signal electrode 212, the resultant charged positive ions and dissociated electrons may then move towards the respective plate 202, 204 or signal electrode 212 having the opposite polarity of the ion/electron. The voltage 206, 208 may be considered to be a polarising, or polarisation, voltage. The pair of plates 202, 204 may be considered to be polarisation plates and/or polarisation electrodes.

The movement of the ion pairs generates an ionisation current (represented with an arrow labelled 'I' in Fig. 2) within the beam detection volume 209. In the example of Fig. 2, the ionisation current is collected at the signal electrode 212 arranged within the beam detection volume 209. In this example, the signal electrode 212 is connected to a dosimetry card or electrometer 214, and properties of the treatment beam 201 can be determined by measuring the ionisation current. For example, the ionisation current should be proportional to the number of ion pairs created, in turn giving an indication of the properties of the ionising radiation. In other examples, different approaches for measuring the ionisation current for dosimetry may be used.

The apparatus 200 may therefore in some examples take the form of an ion chamber. In some examples, the pair of plates 202, 204, the signal electrode 212, the beam detection volume 209, and the voltage sensor 210 are each at least partly arranged within a sealed chamber, which optionally may itself be at least partially enclosed by the plates 202, 204. For example, a chamber or enclosure may be formed between the plates 202, 204 and a sidewall component, and the chamber then provided with gas within and sealed. The interior of the chamber may effectively correspond to the beam detection volume 209, but need not necessarily do so.

The apparatus 200 also comprises a voltage sensor 210 arranged on a surface of at least one of the pair of plates 202, 204 and/or the signal electrode 212, wherein the voltage sensor 210 is arranged to sense, determine, measure, and/or detect a voltage generated between the plates 202, 204 and the signal electrode 212. In other words, the voltage sensor 210 is arranged to sense a voltage generated across at least part of the beam detection volume 209. The voltage sensor 210 may be arranged on one of the opposing surfaces of the pair of plates 202, 204. For example, the voltage sensor 210 may be located at least in part on a top surface of the bottom plate 204, as shown in Fig. 2. In other examples, the voltage sensor 210 may be located at least in part on the underneath surface of the top plate 202. It will be appreciated that other variations are possible. In some examples, the voltage sensor 210 is located entirely on a particular one of the pair of plates 202, 204. In some examples, the voltage sensor 210 is located at least in part on a surface of the signal electrode 212.

Advantageously, the voltage sensor 210 being thus arranged on a surface of a plate 202, 204 and/or signal electrode 212 enables the voltage sensor 210 to provide an indication of the voltage generated between the plates 202, 204 and the signal electrode 212 without requiring any further space than is already being used for the beam detection volume 209. Accurate determination of the voltage generated in the beam detection volume consequently enables improved monitoring of the radiotherapy treatment beam dose, which in turn can enable improved delivery of treatment. Accordingly, the apparatus 200 can provide a dose measurement device that is both reliable and compact in size compared to conventional ion chambers that have associated external electrical or electronic components. Such a device is particularly beneficial for integration into smaller radiotherapy treatment heads and facilitates the reduction in size of the overall treatment head.

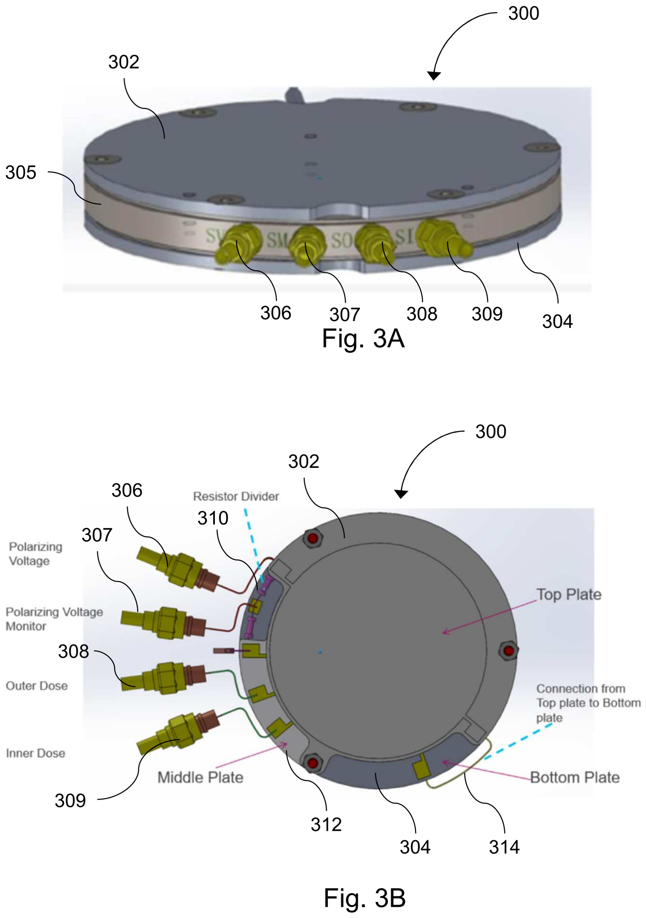

A particular configuration of two plates 202, 204 and a signal electrode (which may be a middle plate) 212 is shown in Figs. 2, 3A, and 3B, such that a total of at least three plate or electrode components are present in the apparatus 200, 300. However, the disclosed apparatus may also be implemented as a more simple configuration based on using two plates, rather than three. In such examples, the two plates 202, 204 are not held at the same voltage and are not electrically connected to each other. Instead, a first plate may be held at a first voltage 206, and the second plate may be held at a negative or ground voltage 208, in order to apply a voltage across a beam detection volume 209 arranged between the two plates. As described above, an ionisation current will be produced between the two plates, and may be detected using any suitable means. A separate signal electrode may be omitted in such examples. Accordingly, each of the examples disclosed herein may be implemented as a two plate implementation rather than the three plate (or two plates plus electrode) implementation shown in Figs. 2, 3A, and 3B. In each example, a beam detection volume is arranged at least in part between a pair of plates and the pair of plates are arranged to generate a voltage across at least part of that beam detection volume.

Accordingly, generally disclosed herein is apparatus for measuring radiotherapy beam dose, the apparatus comprising: a pair of plates for generating a voltage between the pair of plates; a beam detection volume arranged at least in part between the pair of plates; and a voltage sensor arranged on a surface of at least one of the pair of plates, wherein the voltage sensor is arranged to sense a voltage generated across at least part of the beam detection volume. In some examples of this general concept, the pair of plates may be considered to correspond to the plates 202, 204, and/or may correspond to one of the plates 202, 204 along with the signal electrode 212, depending upon the specific implementation.

In some examples, the voltage sensor 210 is arranged towards an outer edge of the surface of the plate 202, 204 and/or signal electrode 212. For example, the voltage sensor 210 may be fixed to the surface of the plate near to a perimeter of the plate or electrode, rather than near to a centre of the plate or electrode, and/or closer to the perimeter of the plate or electrode than the centre. As the apparatus 200 will typically be aligned to measure the peak radiation of the beam at the centre of the plates, advantageously, placing the voltage sensor 210 towards an outer edge of the surface of the plate and/or electrode may reduce or greatly reduce the amount of radiation that the voltage sensor is exposed to, and can thus prolong the lifetime of the apparatus.

In some examples, the voltage sensor 210 is arranged within 10 millimetres (mm) of the outer edge, or perimeter, of the surface of the plate or electrode. In some examples, the voltage sensor 210 is arranged within 5 mm of the outer edge, or perimeter, of the surface of the plate or electrode. In some examples, the voltage sensor 210 is arranged at a distance of 1.3 mm from the outer edge, or perimeter, of the surface of the plate or electrode, such that a distance between a particular point on the voltage sensor 210 and the outer edge or perimeter of the surface of the plate or electrode is 1.3 mm.

In some examples, the voltage sensor 210 comprises a plurality of resistors. Placing resistors within the beam detection volume is contrary to conventional approaches of providing an ion chamber with electrical or electronic components mounted externally to the chamber itself. In some examples, the voltage sensor 210 comprises a potential divider, which may be a circuit formed by resistors and/or similar components. The voltage sensor 210 may thus sample the polarising voltage V being applied across the beam detection volume 209. In some examples, the voltage sensor 210 is soldered to the surface of the plate and/or signal electrode 212 on which it is arranged. For example, multiple resistors may be soldered to the surface of at least one of the plates 202, 204 and/or signal electrode 212 to form a potential divider for measuring the voltage generated across at least part of the beam detection volume 209 by the plates 202, 204 and signal electrode 212.

In some examples, such as those in which a resistor and/or potential divider is used, the voltage sensor 210 comprises at least one piezoresistor. Advantageously, piezoresistors may be particularly small in size and may therefore be positioned further away from the centre of the radiation beam or treatment beam 201.

In some examples, the voltage sensor 210 comprises at least one metal film resistor. Advantageously, a metal film resistor may be particularly robust in a radiation environment, and particular types of metal film resistor may be particularly radiation hard, thereby enabling improved long-term reliability of the apparatus 200.

In some examples, the pair of plates 202, 204, the beam detection volume 209, and the voltage sensor 210 are arranged within a sealed chamber. In such examples, the sealed chamber is optionally sealed at least in part with at least one of: laser soldering, indium silk solder, and/or a hard solder. Advantageously, providing a sealed chamber can remove the need for some of the auxiliary electronics associated with unsealed ion chambers, thereby enabling a further reduction in the overall size of the apparatus 200.

As described above, in some examples the beam detection volume contains ionisable gas. In some examples, the voltage sensor 210 is arranged within the beam detection volume 209, but need not necessarily be so. For example, the voltage sensor 210 may be arranged adjacent to the beam detection volume 209.

For some implementations, apparatus 200 as shown in Fig. 2 may provide a first device for measuring radiotherapy beam dose, and a second device for measuring radiotherapy beam dose may also be provided and connected to the first device such that two measurements of the energy or dose of the treatment beam 201 can be taken. In such examples, the apparatus as shown in Fig. 2 may be used as a primary beam monitor and a duplicate or near-duplicate second device may be used as a secondary beam monitor.

For example, a further set of the components shown in Fig. 2 may be provided stacked on top of or below the apparatus 200 as shown in Fig. 2. In such examples, the apparatus comprises a second pair of plates for generating a voltage between the second pair of plates; a second beam detection volume arranged at least in part between the second pair of plates; and a second voltage sensor arranged on a surface of at least one of the second pair of plates, wherein the second voltage sensor is arranged to determine a voltage generated across at least part of the beam detection volume. The second beam detection volume, second pair of plates, and second voltage sensor may each be arranged according to any of the examples of the beam detection volume 209, the pair of plates 202, 204, and the voltage sensor 210 disclosed herein (including corresponding components in Figs. 3A and 3B). In some examples, the first voltage sensor of the first device of the apparatus 200 and the second voltage sensor of the second device of the apparatus 200 are arranged to verify that the two devices are correctly connected. For example, each voltage sensor may comprise a resistor-based potential divider, and each voltage sensor may use different resistor values to the other voltage sensor such that an output of each voltage sensor is different for a given input voltage, thereby facilitating determination of whether the two devices are correctly connected.

In some implementations, the apparatus 200 may be mountable, or attachable, to a treatment head of a radiotherapy system. In some examples, the apparatus 200 may be integrated within the treatment head.

Fig. 3A shows a perspective view of an apparatus 300 for measuring radiotherapy beam dose according to the present disclosure. The apparatus 300 is based on the apparatus 200 of Fig. 2 and is a particular implementation of the apparatus 200 of Fig 2. It will thus be understood that each feature described below in relation to the apparatus 300 may also be used with particular examples of the apparatus 200.

In the example of Fig. 3A, a pair of plates 302, 304, along with a sidewall 305, enclose a beam detection volume (not shown). Although the plates of Fig. 3 are shown as circular, it will be appreciated that other shapes of plate may be used in any of the examples disclosed herein, including square plates and/or rectangular plates. The plates 302, 304 correspond to the plates 202, 204 of Fig. 2. In the perspective of Fig. 3A, the apparatus 300 is sealed to form a sealed gas-filled chamber corresponding to the beam detection volume, within which is a voltage sensor like the voltage sensor 210 of Fig. 2. The apparatus 300 is arranged to produce a detectable ionisation current as described above in relation to Fig. 2, and thereby provide radiotherapy dosimetry.

Fig. 3B shows another perspective view of the apparatus 300 for measuring radiotherapy beam dose according to the present disclosure. In Fig. 3B, the apparatus 300 is viewed from above the top plate 302 of the pair of plates 302, 304. However, partial cutouts of the top plate 302 are shown such that parts of the bottom plate 304 can be seen and part of a signal electrode 312 can be seen as a middle plate, the signal electrode 312 being for collecting ionisation current as described above in relation to the example of Fig. 2. As described above, the signal electrode 312 may be connected to ground.

Through a cutout of the top plate 302, voltage sensor 310 is shown attached to a surface of the bottom plate 304. In this example, the voltage sensor 310 is a resistor-based potential divider. The voltage sensor 310 components in this example are arranged as a potential divider in relation to wiring connecting the polarising voltage connector 306 to the plate 304.

Also shown in Fig. 3B is a connection 314 from the top plate to the bottom plate ensuring that those plates may be held at the same voltage, such as the positive voltage 206 in Fig. 2.

The apparatus 300 of Figs. 3A and 3B also comprises four connectors 306-309. The connectors 306-309 are electrical connectors for supplying and/or obtaining electrical signals to/from the apparatus 300. Each connector is shown as a coaxial SMA (SubMiniature version A) connector, although need not necessarily be so, and in other examples, other types of connector may be used, including other types of coaxial connector. A first connector 306 is arranged for providing the (polarising) voltage to the pair of plates. A second connector 307 is arranged for collecting signals from the voltage sensor 310 to enable monitoring of the polarising voltage. As shown in Fig. 3B, the second connector 307 is thus connected to the voltage sensor 310.

A third connector 308 is arranged for making a first ionisation current measurement (and thereby, a beam dose and/or energy measurement) from a signal electrode. A fourth connector 309 is arranged for making a second ionisation current measurement from a signal electrode. The first ionisation current measurement and the second ionisation current measurement may respectively correspond to an outer dose measurement and an inner dose measurement, with the apparatus 300 being arranged with an outer section for measurement of dose towards the outside of the beam cross-section and an inner section for measurement of dose towards the centre of the beam cross-section.

An internal interface of each of the four connectors may be connected to the relevant corresponding part of the apparatus 300 by using any suitable electrical or electronic approach, such as wiring and/or soldering. An external interface of each of the four connectors may then provide an external connection to/from the apparatus 300 for obtaining and/or providing the respective signal, such as by using external coaxial cabling and/or wiring. Accordingly, each connector can provide an electrical interface between the apparatus 300 and a radiotherapy system and/or a controller for a radiotherapy system. Advantageously, said electrical interface is relatively small in size, and may be particularly small if small SMA connectors are used. It will be appreciated that it is not always necessary to use four connectors and in other examples, a different number of connectors may be used.

Hence, in some examples, the apparatuses 200, 300 comprise a coaxial connector for transmitting signals from the voltage sensor, such as an SMA connector, and which may correspond to the second connector 307 in Figs. 3A and 3B.

In some examples, the coaxial connector, or any other connectors, may comprise glass insulation. The glass insulation can provide an electrical insulation to the connector. Advantageously, the glass insulation may provide improved irradiation endurance compared to conventional tetrafluroethylene insulation.

Although the connectors 306-309 are shown as detached from the main body of the apparatus 300 in Fig. 3B, for use, part of those connectors can be inserted into the main body of the apparatus 300 and soldered in place to provide a leak-proof seal, as shown in Fig. 3A.

In some examples, a surface of the coaxial connector comprises Kovar alloy. For example, a metal surface of a socket of the connector that is inserted into the sidewall 305 may comprise a Kovar alloy. The Kovar alloy may advantageously improve the seal between the connector and the sidewall 305 (or, in other examples, any other part of the body of the apparatus 300) when solder is used to seal the interface between the connector and the sidewall 305, and thus enable a reduced gas leakage rate from the apparatus.

In some examples, 306 stainless steel is used to form part of the body of the apparatus 200, 300, such as the sidewall 305. Low temperature solder can be used for solder between the Kovar alloy on a connector and the 306 stainless steel, and high temperature solder can be used to solder the 306 stainless steel elsewhere. The apparatus 200, 300 can therefore be sealed using a method comprising a first step of using a high temperature solder to seal the body components of the apparatus to form a sealed chamber (such as by soldering the sidewall 305 to the plates 302, 304), and a second step of using low temperature solder to seal at least one interface between a component of the body of the apparatus and a connector socket, the connector socket comprising Kovar alloy.

Hence, in some examples, the apparatus comprises a coaxial connector for transmitting signals from the voltage sensor and an interface between the coaxial connector and a body of the apparatus is sealed by using a low temperature solder. Optionally, the body of the apparatus (which may comprise the plates 302, 304 and the sidewall 305) may comprise a sealed interface that is sealed by using high temperature solder.

Various forms of low temperature solder and high temperature solder material will be known to those skilled in the art. Those skilled in the art will understand that "low temperature" and "high temperature" refer to the melting points of the solder material used in the soldering process. In some examples, a low temperature solder may be considered to be a material with a melting point ranging from around 50°C to 160°C, such as alloys of tin, whereas a high temperature solder may be considered to be a material with a higher melting point, such as alloys of lead.

Each connector of the apparatus 200, 300 may be arranged according to any one or more of the examples disclosed herein.

Like the apparatus 200 of Fig. 2, the apparatus 300 of Figs. 3A and 3B may be arranged to be mounted and/or attached to a treatment head of a radiotherapy system. Also provided herein is a radiotherapy system comprising one or more of the apparatuses 200, 300 described herein.

In a particular example, by following the approaches disclosed herein, an apparatus 200, 300 for measuring radiotherapy beam dose may be provided that fits within a footprint having dimensions of approximately 154 mm x 34 mm, with a disc-like shape having a diameter of approximately 154 mm and a height or thickness of 34 mm. In some examples, the dimensions of the apparatus 200, 300 may be approximately 124 mm x 17 mm, with a disc-like shape having a diameter of approximately 124 mm and a height or thickness of 17 mm. In each example, the diameter of the device may correspond approximately to the diameter of the plates used, with that compact size being based on providing the voltage sensor on a surface of a plate or signal electrode as described herein and therefore avoiding the need for sizeable external auxiliary components. Such apparatus is advantageously highly compact compared to conventional ion chambers for radiotherapy systems.

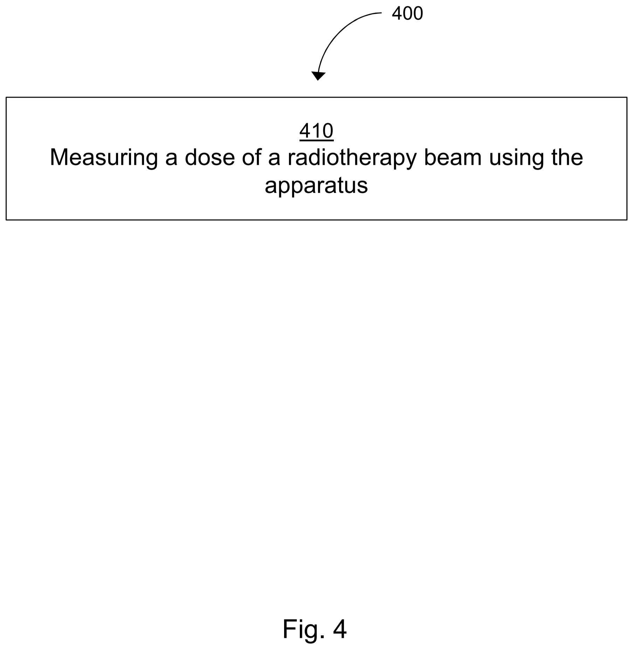

Fig. 4 shows a flowchart of a method 400 for measuring radiotherapy beam dose according to the present disclosure. The method 400 comprising measuring 410 a dose of a radiotherapy beam using the apparatuses 200, 300 disclosed herein or a radiotherapy system comprising those apparatuses. The method 400 may be performed without a patient present, such as during calibration, set-up, maintenance, and/or installation of the radiotherapy system.

A computer-based system may be used for controlling or operating various parts of the systems, devices, methods and apparatuses disclosed herein. The computer-based system can be implemented in software, firmware and/or hardware and may comprise a computer-readable medium containing instructions that, when executed by a processor, cause the system to perform any of the methods described herein.

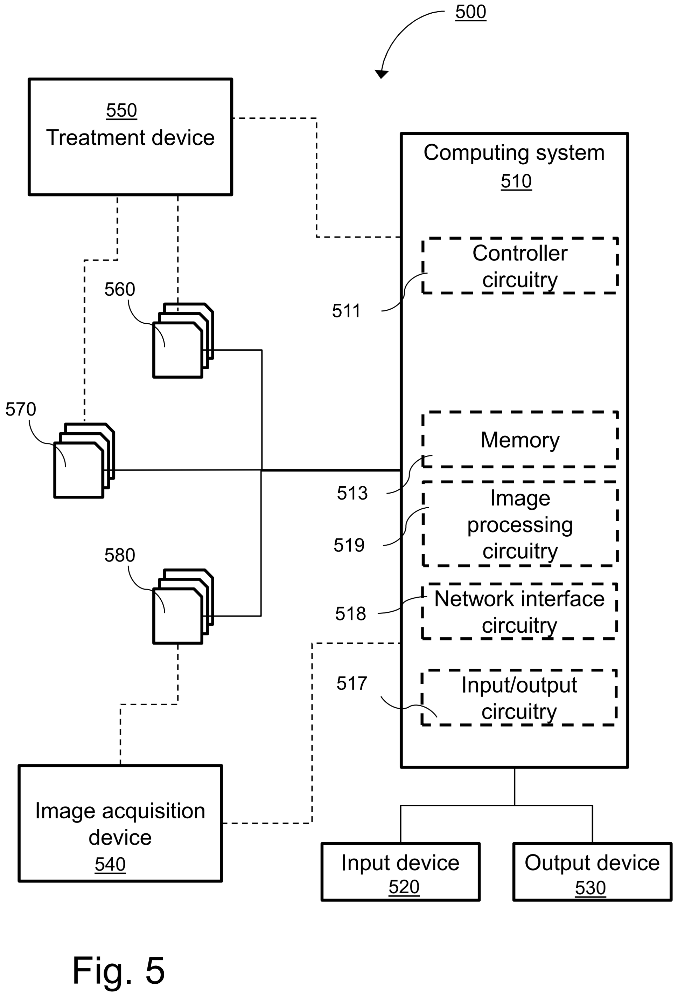

Fig. 5 illustrates a block diagram of one implementation of a radiotherapy system 500. The radiotherapy system 500 comprises a computing system 510 within which a set of instructions, for causing the computing system 510 to perform any one or more of the methods discussed herein, may be executed.

The computing system 510 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 510 includes controller circuitry 511 and a memory 513 (e.g., read-only memory (ROM), flash memory, dynamic random access memory (DRAM) such as synchronous DRAM (SDRAM) or Rambus DRAM (RDRAM), etc.). The memory 513 may comprise a static memory (e.g., flash memory, static random access memory (SRAM), etc.), and/or a secondary memory (e.g., a data storage device), which communicate with each other via a bus (not shown).

Controller circuitry 511 represents one or more general-purpose processors such as a microprocessor, central processing unit, accelerated processing units, or the like. More particularly, the controller circuitry 511 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 511 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 511 is configured to execute the processing logic for performing the operations and steps discussed herein.

The computing system 510 may further include a network interface circuitry 518. The computing system 510 may be communicatively coupled to an input device 520 and/or an output device 530, via input/output circuitry 517. In some implementations, the input device 520 and/or the output device 530 may be elements of the computing system 510. The input device 520 may include an alphanumeric input device (e.g., a keyboard or touchscreen), a cursor control device (e.g., a mouse or touchscreen), an audio device such as a microphone, and/or a haptic input device. The output device 530 may include an audio device such as a speaker, a video display unit (e.g., a liquid crystal display (LCD) or a cathode ray tube (CRT)), and/or a haptic output device. In some implementations, the input device 520 and the output device 530 may be provided as a single device, or as separate devices.

In some implementations, the computing system 510 may comprise image processing circuitry 519. Image processing circuitry 519 may be configured to process image data 580 (e.g. images, or imaging data), such as medical images obtained from one or more imaging data sources, a treatment device 550 and/or an image acquisition device 540. Image processing circuitry 519 may be configured to process, or pre-process, image data. For example, image processing circuitry 519 may convert received image data into a particular format, size, resolution or the like. In some implementations, image processing circuitry 519 may be combined with controller circuitry 511.

In some implementations, the radiotherapy system 500 may further comprise an image acquisition device 540 and/or a treatment device 550, such as those disclosed herein in the example of Fig. 1. The image acquisition device 540 and the treatment device 550 may be provided as a single device. In some implementations, treatment device 550 is configured to perform imaging, for example in addition to providing treatment and/or during treatment. The treatment device 550 comprises the main radiation delivery components of the radiotherapy system, such as the beam generation systems and linear accelerator components disclosed herein.

Image acquisition device 540 may be configured to perform positron emission tomography (PET), computed tomography (CT), and magnetic resonance imaging (MRI).

Image acquisition device 540 may be configured to output image data 580, which may be accessed by computing system 510. Treatment device 550 may be configured to output treatment data 560, which may be accessed by computing system 510.

Computing system 510 may be configured to access or obtain treatment data 560, planning data 570 and/or image data 580. Treatment data 560 may be obtained from an internal data source (e.g. from memory 513) or from an external data source, such as treatment device 550 or an external database. Planning data 570 may be obtained from memory 513 and/or from an external source, such as a planning database. Planning data 570 may comprise information obtained from one or more of the image acquisition device 540 and the treatment device 550.

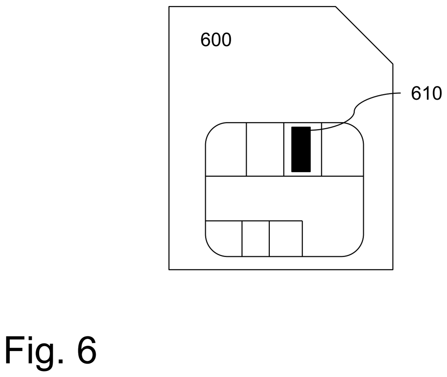

The various methods described above may be implemented by a computer program. The computer program may include computer code (e.g. instructions) 610 arranged to instruct a computer to perform the functions of one or more of the various methods described above. The steps of the methods described above may be performed in any suitable order. The computer program and/or the code 610 for performing such methods may be provided to an apparatus, such as a computer, on one or more computer readable media or, more generally, a computer program product 600)), depicted in Fig. 6. The computer readable media may be transitory or non-transitory. The one or more computer readable media 600 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 610 may also reside, completely or at least partially, within the memory 513 and/or within the controller circuitry 511 during execution thereof by the computing system 510, the memory 513 and the controller circuitry 511 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. Apparatus for measuring radiotherapy beam dose, the apparatus comprising:
a pair of plates for generating a voltage between the pair of plates;
a beam detection volume arranged at least in part between the pair of plates; and
a voltage sensor arranged on a surface of at least one of the pair of plates, wherein the voltage sensor is arranged to sense a voltage generated across at least part of the beam detection volume.

2. The apparatus of claim 1, wherein the voltage sensor is arranged towards an outer edge of the surface of the plate.

3. The apparatus of any preceding claim, wherein the voltage sensor comprises a potential divider and/or a plurality of resistors.

4. The apparatus of any preceding claim, wherein the voltage sensor is soldered to the surface of the plate.

5. The apparatus of any preceding claim, wherein the pair of plates, the beam detection volume, and the voltage sensor are arranged within a sealed chamber, optionally wherein the sealed chamber is sealed at least in part with at least one of: laser soldering, indium silk solder, and/or a hard solder.

6. The apparatus of any preceding claim, further comprising:
a second pair of plates for generating a voltage between the second pair of plates;
a second beam detection volume arranged at least in part between the second pair of plates; and
a second voltage sensor arranged on a surface of at least one of the second pair of plates, wherein the second voltage sensor is arranged to sense a voltage generated across at least part of the second beam detection volume.

7. The apparatus of any preceding claim, wherein the beam detection volume contains ionisable gas.

8. The apparatus of any preceding claim, wherein the voltage sensor comprises at least one piezoresistor and/or wherein the voltage sensor comprises at least one metal film resistor.

9. The apparatus of any preceding claim, wherein the apparatus further comprises a coaxial connector for transmitting signals from the voltage sensor, optionally wherein the coaxial connector comprises glass insulation.

10. The apparatus of claim 9, wherein a surface of the coaxial connector comprises Kovar alloy.

11. The apparatus of any of claims 9 to 10, wherein an interface between the coaxial connector and a body of the apparatus is sealed using a low temperature solder.

12. The apparatus of claim 11, wherein the body of the apparatus comprises a sealed interface that is sealed using a high temperature solder.

13. The apparatus of any preceding claim, wherein the voltage sensor is arranged within the beam detection volume.

14. A method for measuring radiotherapy beam dose, the method comprising measuring a dose of a radiotherapy beam using the apparatus of any of claims 1 to 13.

15. A computer-readable medium containing instructions that, when executed by a processor, cause the method of claim 14 to be performed.
